# DEMANDE DE BREVET EUROPEEN

(11) **EP 0 713 092 A1**
(43) Date de publication de la demande: **22.05.1996**
(21) Numéro de dépôt: 95402526.8
(22) Date de dépôt: 10.11.1995
(51) Int. Cl.: G01N 33/50, C12N 5/00

(54) **Procédé de criblage de facteurs sur modèle de paralysie et méthode d'obtention de ce modèle**

(30) Priorité: 21.11.1994 FR 9413905
(71) Demandeur: Feldblum, Sophie, F-75015 Paris (FR)
(72) Inventeur: Feldblum, Sophie, F-75015 Paris (FR)
(74) Mandataire: Bernasconi, Jean

(57) **Abrégé**

La méthode permet d'induire chez l'animal une paralysie, de préférence paraplégie, complète et réversible en une durée prédéterminée à l'aide d'un ballon venant comprimr la moelle épinière. Le modèle animal est mis en oeuvre dans un procédé de test de facteurs-candidats à visée neuroprotectrice et/ou neurorégénératrice, qui sont considérés comme positifs si l'on peut observer une réduction de la durée de la paralysie. Médicaments, notamment neuroprotecteurs et/ou neurorégénérateurs, comprenant un principe actif révélé par le procédé de test.

## Description

La présente invention a trait à un procédé de test ou de criblage de facteurs à visée neuroprotectrice ou de régénération sur un modèle animal de paralysie, de préférence paraplégie, induite. Elle concerne également la méthode permettant d'induire une paralysie, notamment paraplégie, chez l'animal ainsi que les facteurs répondant positivement au procédé de criblage.

Afin d'étudier la pathophysiologie des lésions compressives de la moelle épinière chez l'homme et rendre possible l'évaluation de méthodes thérapeutiques de ces lésions, des auteurs se sont penchés depuis fort longtemps sur la mise au point de méthodes visant à provoquer artificiellement des lésions médullaires dans le dessein d'induire des paralysies ou des paraplégies complètes et permanentes chez l'animal.

Dès 1953, I.M. TARLOV et al. (A.M.A. Archives of Neurology and Psychiatry, volume 70, pages 813-819) décrivaient un dispositif permettant de réaliser chez le chien éveillé une compression médullaire graduelle, par la mise en place dans l'espace sous-dural d'un ballon dont l'intensité de glonflement déterminait le degré de compression. Les auteurs conclurent simplement qu'il est possible d'obtenir une paralysie complète.

Le principe de la compression par ballon a été repris en 1973 par C.H. TATOR (The Canadian Journal of Surgery, Volume 16, pages 222 à 231) et appliqué au singe. Le moyen de compression est un ballon annulaire qui se place autour de la moelle épinière dans l'espace extradural. Dans les conditions d'essai, une paraplégie complète immédiate (observée à 1 semaine) est obtenue à partir de 250 mmHg pendant 5 min, suivie d'une récupération partielle observable à partir de la quatrième semaine. Dans un autre essai, pour un même degré de compression (350 mmHg) pendant 5 min, sur 10 singes paralysés, la moitié restait paraplégique au bout de 12 semaines, tandis que l'autre moitié ne présentait qu'une récupération partielle à partir de la quatrième semaine.

En 1992, D. MARTIN et al. (Journal of Neuroscience Research 32: 539 à 550) ont adapté au rat le modèle de compression par ballon en utilisant des ballons GV15 (Ingénor, Paris) gonflés par des volumes de fluide allant de 10 à 100 µl à l'aide d'une seringue Hamilton et par l'intermédiaire d'un cathéter. Les animaux sont anesthésiés par l'injection intrapéritonéale d'un mélange de Xylazine et de Ketalar. Après incision de la peau, les muscles paravertébraux sont détachés et une laminectomie est réalisée au niveau T8, T9 ou T10. La dure-mère est ouverte et le ballon est introduit dans l'espace sous-arachnoïdien et déplacé rostralement d'une valeur d'une ou deux vertèbres au-dessus de la laminectomie. Le degré de compression est essentiellement fonction, là encore, du volume de liquide injecté dans un ballon donné. Les observations sont faites à des intervalles d'une semaine. Pour une durée de compression de 5 min, le seuil de volume était de 40 µl pour obtenir une paraplégie complète et définitive, ce qui était l'objectif recherché. Pour les faibles degrés de compression, les auteurs ont seulement observé un déficit moteur réduit et transitoire (10 µl) sur l'échelle de Tarlov ou des paraparesies transitoires d'ampleur variable (20 et 30 µl), avec récupération motrice totale après 4 semaines (voir aussi D. MARTIN et al., Brain Research Bulletin, volume 30, pages 507 à 514, 1993).

Contrairement à l'enseignement de ces documents, l'inventeur a découvert de manière inattendue et surprenante qu'il était possible d'induire, très simplement et de manière parfaitement reproductible, une paraplégie complète de courte durée, notamment de l'ordre d'une semaine environ, chez un animal tel que le rat et que cette méthode permettait de mettre au point un modèle animal pour le criblage rapide de facteurs en vue d'étudier leurs potentialités comme promoteurs de la récupération. Cette découverte peut être étendue à l'induction de paralysies de type tétraplégie de courte durée, comme à l'induction de paralysies, notamment de paraplégies, de durées variées.

L'invention rend possible pour la première fois le criblage de facteurs sur un modèle de paralysie, de préférence paraplégie, complète et réversible et qui plus est de durée standardisée. Elle a donc pour objet un procédé de test ou de criblage de facteurs-candidats à visée neuroprotectrice et/ou de neurorégénération, dans lequel on administre le facteur-candidat à un animal présentant une paralysie complète mais réversible de durée prédéterminée et l'on détecte la capacité de cette substance à réduire la durée de la paralysie. Le procédé est particulièrement remarquable par le fait qu'il est le premier à ce jour à permettre d'étudier directement l'action d'un facteur-candidat sur une phase de récupération naturelle de paralysie, notamment paraplégie.

Par facteurs neuroprotecteurs, on entend notamment les facteurs capables de limiter la lésion initiale, tandis que par facteurs neurorégénérateurs, on entend notamment les facteurs susceptibles de stimuler la repousse axonale et de faciliter ainsi la restructuration de la zone lésée. Ces deux types de facteurs peuvent être regroupés sous la définition de promoteurs de la récupération. Les facteurs incluent des substances chimiques et des cellules ou des cellules de lignée cellulaire susceptibles d'être greffées.

Préférentiellement appliquée à des animaux, en particulier des rats, présentant une paralysie, de préférence paraplégie, complète de courte durée selon l'invention, le procédé de test de facteurs est remarquable par sa grande rapidité; le modèle préféré est celui de la paraplégie réversible en une moyenne de 6 jours environ (voir le tableau dans la partie Résultats ci-après). La fin de la paralysie, ici la paraplégie, est définie par une récupération motrice partielle correspondant à l'observation d'un début de marche. Si l'on se base sur la récupération motrice totale (marche normale et faculté de se redresser), ce modèle préféré atteint ce dernier stade en une moyenne de 9 jours environ.

Selon l'invention, le facteur candidat pourra être administré à l'animal avant ou après la compression et de préférence à faible intervalle de temps par rapport à cette compression, ce qui peut aller notamment de quelques minutes avant la compression à quelques minutes ou heures après la compression. Préférentiellement, le facteur sera administré environ 1 heure après la compression. Dans le cas de facteurs à potentiel régénérateur, il pourra être administré plus tardivement, sans dépasser généralement les 3ème à 5ème jours post-compression. Les facteurs sont de préférence administrés par injection; les cellules ou cellules de lignée cellulaire sont greffées, notamment injectées, au site de compression.

L'invention concerne notamment un procédé de test ou de criblage de tels facteurs, dans lequel on induit chez l'animal, en particulier le rat, une paralysie, de préférence paraplégie, complète mais réversible de durée prédéterminée, notamment d'une moyenne de 6 jours environ, par compression de la moelle épinière de l'animal anesthésié, à l'aide d'un ballon, et dans lequel, avant, pendant ou après la compression, on administre à l'animal le facteur à tester, puis l'on détecte la capacité de ce facteur à réduire la durée de la paralysie, notamment paraplégie.

L'induction de la paralysie, notamment paraplégie, réversible dans le cadre de ce procédé de test est de préférence réalisée comme cela est décrit ci-après à propos de la méthode destinée à induire une paralysie, notamment paraplégie, conforme à l'invention.

La présente invention a donc aussi pour objet la méthode permettant d'induire chez l'animal, en particulier le rat, une paralysie, de préférence paraplégie, complète et naturellement réversible, comprenant les étapes de placer un ballon au contact de la moelle épinière, de gonfler le ballon de manière à comprimer temporairement la moelle épinière dans des conditions conduisant à l'obtention d'une paralysie, de préférence paraplégie, complète et réversible de durée prédéterminée, puis de dégonfler et retirer le ballon.

De préférence, le ballon est inséré dans l'espace sous-dural. Pour l'obtention d'une paraplégie selon l'invention, le ballon peut être inséré au niveau thoracique bas ou haut, de préférence thoracique bas, ou au niveau lombaire, notamment lombaire bas; on préférera l'insertion au niveau thoracique bas allant de T6 à T13, et par exemple au niveau T9-T10. Pour l'obtention d'une tétraplégie, l'insertion pourra se faire au niveau cervical.

On utilise de préférence un ballon conçu pour induire une paraplégie réversible en une moyenne de 6 jours environ sur des animaux anesthésiés au moyen d'un anesthésique ayant des propriétés neuroprotectrices. Selon l'invention, les anesthésiques de ce type sont notamment choisis parmi le groupe consistant en Kétamine (appartient à la famille des anesthésiques fixes dissociants), Xylazine (une benzodiazépine) ou mélange de ceux-ci. Un anesthésique préféré est un mélange de Kétamine et de Xylazine. D'autres anesthésiques adaptés sont indiqués plus loin en liaison avec l'animal servant de modèle.

Le modèle-type de ballon pour réaliser l'invention est un ballon de type Goldvalve GV15 d'Ingénor Paris, qui, à sa capacité maximale, peut contenir un volume de 100 µl et présente une longueur de 8 mm et un diamètre de 6 mm.

Dans un mode de réalisation préféré de l'invention, la méthode pour réaliser une paraplégie réversible en une moyenne de 6 jours environ (voir le tableau dans la partie Résultats ci-après), comprend l'injection, dans le ballon, d'un volume de 10 µl environ sur des animaux anesthésiés au moyen d'un anesthésique tel que décrit ci-dessus.

Ce type d'anesthésique a pour propriété de raccourcir la durée de récupération motrice. L'utilisation d'anesthésique sans propriété neuroprotectrice, tel que l'anesthésique Equithezine, conduit dans les mêmes conditions de volume à une durée de récupération supérieure mais restant dans le cadre de l'invention, notamment 10 jours en moyenne pour la récupération motrice partielle (voir le tableau dans la partie Résultats) et environ 16 jours en moyenne pour une récupération motrice totale.

On peut également faire varier la durée de la paraplégie ou de la tétraplégie en modifiant le volume injecté dans le ballon. L'invention porte également sur l'utilisation de volumes de gonflement situés autour desdit 10 µl tout en restant de préférence au-dessous des 20 µl.

Il est bien évident qu'à partir du modèle-type de ballon indiqué et des conditions de son utilisation, le spécialiste est à même de choisir et d'essayer d'autres ballons et de déterminer pour eux, par exemple par l'essai, les paramètres appropriés sans sortir du cadre de l'invention.

On a présenté ci-dessus des modèles particuliers de paraplégie réversible en 6 et 10 jours environ. Il va de soi bien évidemment que l'invention permet à l'homme du métier de créer des modèles de durées de paraplégie ou de tétraplégie différentes, notamment en jouant sur les caractéristiques des ballons et le volume injecté.

De préférence, le ballon est placé de manière médiane par rapport à la moelle épinière afin d'obtenir une paralysie, notamment paraplégie, bilatérale. Toutefois, l'invention permet également de réaliser une paralysie unilatérale en plaçant le ballon latéralement par rapport à la moelle épinière. On obtient ainsi un animal conservant une motricité unilatérale.

Enfin, si le modèle animal décrit en détail ici est le rat, l'invention couvre aussi l'emploi d'autres animaux tels que notamment le chat, le chien et le singe. Les conditions opératoires pourront être adaptées à l'animal choisi à l'aide d'essais basés sur les principes établis dans cette demande. Des anesthésiques utilisables sont indiqués plus loin pour chaque animal.

L'invention a encore pour objet :
- tout médicament neuroprotecteur et/ou neurorégénérateur dont le principe actif a été révélé par le procédé du test; ces principes actifs comprennent aussi bien des substances chimiques que des cellules ou cellules de lignée cellulaire;
- des médicaments comprenant des substances, molécules, cellules ou des cellules de lignée cellulaire jamais utilisées à titre de médicament et répondant positivement au procédé de test;
- l'utilisation de substances, molécules, cellules ou cellules de lignée cellulaire connues en thérapeutique et répondant positivement au test, pour la préparation de médicaments neuroprotecteurs et/ou neurorégénérateurs.

En plus du principe actif, ces médicaments peuvent comprendre, comme cela est d'usage, des excipients pharmarceutiques acceptables.

L'invention va être maintenant décrite plus en détail dans ce qui suit, notamment par l'indication, à titre d'exemple non limitatif, d'un mode d'induction de paraplégies réversibles de durées moyennes de 6 et 10 jours respectivement.

### Matériel utilisé :

La lésion médullaire est réalisée à l'aide d'un ballon gonflable initialement destiné à l'occlusion de malformations vasculaires intracrâniennes (ballon Golvalve GV15, Ingénor, Paris). A sa capacité maximale, le ballon peut contenir un volume de 100 µl et présente une longueur de 8 mm et un diamètre de 6 mm. En vue de l'implantation du ballon dans l'espace sous-dural, celui-ci est relié par un cathéter de polyéthylène de diamètre extérieur 0,96 mm (Biotrol) lui même connecté à une embase (BALT) raccordée à un robinet à 3 voies qui est lui même relié à une seringue de 1 ml.

Pour chaque nouveau ballon, on peut effectuer un étalonnage en gonflant le ballon à l'aide d'une seringue Hamilton d'une capacité de 50 µl. Des empreintes de la taille du ballon pour des gonflements de 10, 20, 30, 40 et 50 µl est obtenue par photo et sont comparées aux empreintes du ballon de référence précédemment utilisé. En cas de divergence, le paramètre d'étalonnage est le volume de gonflement qui peut donc légèrement varier pour ce type de ballon autour des 10 µl.

### Méthode :

L'implantation et le gonflement du ballon est réalisé sur des animaux (rattes Sprague-Dawley, Iffa Credo) anesthésiés par une injection intramusculaire d'un mélange de Xylazine (15 mg/kg) (Rompun) et de Kétamine (100 mg/kg) (Imalgène) ou bien d'Equithesine. Après ouverture de la peau, les muscles paravertébraux sont désinsérés des arcs postérieurs des vertèbres. Une laminectomie d'une vertèbre au niveau thoracique T9-T10 est réalisée afin de mettre à nu la moelle épinière. La dure-mère est ouverte à l'aide d'une aiguille 25 G. Le ballon dégonflé est alors inséré par cet orifice, rostralement à la laminectomie. Le gonflement du ballon est réalisé sous des lames intactes. La durée et le degré de compression peuvent être modulés selon le type de gravité recherché. Afin d'obtenir une paraplégie complète totallement réversible en une moyenne de 9 jours environ (récupération motrice totale), il a été réalisé un gonflement correspondant à l'injection de 10 µl d'eau distillée pendant 5 min. Le ballon est ensuite dégonflé puis retiré et la plaie est suturée. Les animaux sont alors placés dans des cages individuelles dans un environnement thermostatisé (28 à 30°C) et sont nourris ad libitum.

### Résultat :

La méthode qui vient d'être décrite a été appliquée à deux groupes de rats, chaque groupe recevant l'un des deux anesthésiques indiqués. Il a été observé dans 100% des cas l'induction d'une paraplégie complète dès le réveil des animaux. Chez les animaux traités avec le mélange Xylazine/Kétamine, l'observation quotidienne de ces animaux a révélé que cet état paraplégique dure en moyenne 6 jours avant de pouvoir observer des mouvements mal coordonnés des membres postérieurs et un début de marche. Il faut attendre encore environ 3 jours de plus pour que ces animaux retrouvent une marche normale et la faculté de se redresser. Cette récupération totale de la motricité a été observée dans 100% des cas. En plus de cette reproductibilité comportementale, l'analyse macroscopique post-mortem de la moelle de ces animaux a montré , au site de compression, une lésion qui s'étend en moyenne sur 1 cm. Cette lésion se caractérise par une dépression correspondant au point d'impact du ballon et par une opacité du tissu. L'analyse microscopique a révélé la présence d'une cavité avec destruction de substance blanche et grise, ainsi qu'une gliose astrocytaire, caractérisée par l'immunodétection de la glial fibrillary acidic protein (GFAP), marqueur spécifique des astrocytes. Ainsi, malgré une récupération motrice complète, une lésion tissulaire importante persiste.

**Tableau:**

| Anesthésique: | Equithesine | Kétamine/Xylazine |
|---|---|---|
| Taille échantillon: | 17 | 19 |
| **Durée moyenne de paraplégie *:** | **10,059** | **5,8947** |
| Déviation standard: | 5,8145 | 3,9143 |
| Erreur standard: | 1,4102 | 0,8980 |

| | | |
|---|---|---|
| * basée sur l'observation d'un début de marche = récupération motrice partielle. | | |

Le test statistique est un test Mann-Whitney:
- borne limite: 81,500
- P = 0,0117 ; cette valeur, inférieure à 0,05, est considérée comme significative.

### Accélération de la récupération motrice :

A partir de ce modèle animal dans lequel la paraplégie persiste pendant une semaine environ, il est possible de rechercher des facteurs susceptibles d'interagir positivement sur cette période de paraplégie en la raccourcissant, afin de proposer des candidats pour induire la récupération de la motricité chez l'humain paraplégique. Ce test de criblage rapide de produits promoteurs de la récupération est particulièrement utile avant de passer au stade ultérieur d'application chez l'humain. Parmi les promoteurs de la récupération, il y a les neuroprotecteurs dont le rôle est de limiter la lésion initiale, et d'autre part les neurorégénérateurs qui sont susceptibles de stimuler la repousse axonale et faciliter ainsi la restructuration de la zone lésée.

Il existe selon l'étape lésionnelle visée, différentes classes de neuroprotecteurs, parmi lesquels les anti-ischémiques, les antagonistes du glutamate, les anticalciques et les antagonistes des radicaux libres. On peut citer notamment les antagonistes non compétitifs du N-Méthyl-D-Aspartate (NMDA) qui sont capables de bloquer la toxicité du glutamate. On peut encore citer des anti-radicaux libres tels que des dérivés des lazaroïdes.

Parmi les neurorégénérateurs, il existe également différentes classes de molécules selon que l'on vise à promouvoir la croissance axonale ou réduire la cicatrice gliale.

Les facteurs nouveaux pourront être testés conformément au procédé selon l'invention. Pour les facteurs répondant positivement au test, leur activité neuroprotectrice ou neurorégénératrice pourra être déterminée ensuite notamment par examen histologique.

Le procédé prévoit de préférence l'utilisation de groupes de rats que l'on soumet à la compression médullaire et auxquels on administre le facteur à tester. L'impact de celle-ci sur la durée de la paraplégie est déterminé par le déplacement de la moyenne des durées de paraplégie (jusqu'à la récupération motrice partielle ou totale) des rats du groupe par rapport à une moyenne-type prédéterminée (par exemple 6 jours) ou par rapport à un groupe témoin traité ayant subi la compression médullaire en parallèle.

Les animaux des groupes de test et des groupes de témoins seront en général sacrifiés, notamment pour réaliser les examens histologiques nécessaires. En l'absence d'examen nécessitant le sacrifice de l'animal, la persistance d'une lésion importante au site de compression conduit aussi à choisir le sacrifice de l'animal.

### Anesthésiques :

Des anesthésiques neuroprotecteurs sont indiqués ci-après, à titre d'exemples non limitatifs, en liaison avec l'animal destiné à servir de modèle conformément à l'invention. L'homme du métier connaît parfaitement ces anesthésiques et leur mode d'utilisation.
- I - CHAT :: Atropine
Xylazine
Analgésiques centraux
Myorelaxants
Kétamine
Midatrène
- II CHIEN :: Atropine
Neuroleptiques
Benzodiazépines
Barbituriques
Neuroleptanalgésie
- III SINGE :: Atropine
Acépromazine
Kétamine
Xylazine
Alfadione

## Revendications

1. Procédé de test de facteurs-candidats à visée neuroprotectrice et/ou neurorégénératrice, dans lequel on administre le facteur-candidat à un animal présentant une paralysie, de préférence paraplégie, complète mais réversible de durée prédéterminée et l'on détecte la capacité de ce facteur à réduire la durée de la paralysie.

2. Procédé de test selon la revendication 1, caractérisé en ce que l'on utilise des rats présentant une paraplégie réversible en une moyenne de 6 jours environ.

3. Procédé de test de facteurs candidats à visée neuroprotectrice et/ou neurorégénératrice, dans lequel on induit chez l'animal, en particulier le rat, une paralysie, de préférence paraplégie, complète mais réversible de durée prédéterminée, par compression de la moelle épinière de l'animal anesthésié, à l'aide d'un ballon, et dans lequel on administre à l'animal le facteur à tester, puis l'on détecte la capacité de ce facteur à réduire la durée de la paralysie.

4. Procédé de test selon la revendication 3, caractérisé en ce que l'on induit chez le rat une paraplégie complète mais réversible en une moyenne de 6 jours environ.

5. Procédé de test selon la revendication 3 ou 4, caractérisé en ce que l'on induit la paralysie, de préférence paraplégie, par compression à l'aide d'un ballon de type Goldvalve GV15 et que l'on gonfle avec environ 10 µl de liquide, notamment pendant 5 min environ.

6. Procédé de test selon l'une quelconque des revendications 3 à 5, caractérisé en ce que le ballon est inséré dans l'espace sous-dural au niveau thoracique bas, par exemple T9-T10.

7. Méthode destinée à induire chez l'animal, en particulier le rat, une paralysie, de préférence paraplégie, complète et réversible, comprenant les étapes de placer un ballon au contact de la moelle épinière, de gonfler le ballon de manière à comprimer temporairement la moelle épinière dans des conditions conduisant à l'obtention d'une paralysie permanente et réversible en une durée prédéterminée, puis de dégonfler et retirer le ballon.

8. Méthode selon la revendication 7, caractérisée en ce que le ballon est inséré dans l'espace sous-dural au niveau thoracique bas, notamment T9-T10.

9. Méthode selon la revendication 7 ou 8, caractérisée en ce que l'on utilise un ballon conçu pour induire une paraplégie réversible en une moyenne de 6 jours environ sur des animaux anesthésiés au moyen d'un anesthésique ayant des propriétés neuroprotectrices.

10. Méthode selon la revendication 9, caractérisée en ce que l'on utilise un ballon de type Goldvalve GV15 avec un volume d'injection de l'ordre de 10 µl.

11. Méthode selon la revendication 10, caractérisée en ce que, au lieu d'un anesthésique à propriété neuroprotectrice, on utilise un anesthésique dépourvu d'une telle propriété et l'on induit une paraplégie réversible en une moyenne de 10 jours environ.

12. Méthode selon l'une quelconque des revendications 6 à 11, caractérisée en ce que le ballon est placé de façon médiane ou latérale par rapport à la moelle épinière.

13. Méthode selon l'une quelconque des revendication 7 à 10, caractérisée en ce que l'animal est anesthésié à l'aide d'un anesthésique à propriété neuroprotectrice.

14. Méthode selon la revendication 13, caractérisée en ce que, comme anesthésique, l'on utilise un mélange de Xylazine et de Kétamine.

15. Médicament neuroprotecteur et/ou neurorégénérateur comprenant un principe actif révélé par le procédé de test selon l'une quelconque des revendications 1 à 6.

16. Médicament comprenant une substance ou des cellules répondant positivement au procédé de test selon l'une quelconque des revendications 1 à 6.

17. Utilisation de substances ou de cellules répondant positivement au test selon l'une quelconque des revendications 1 à 6 pour la préparation de médicaments neuroprotecteurs et/ou neurorégénérateurs.
